# EUROPÄISCHE PATENTSCHRIFT

(11) **EP 3 968 008 B1**
(45) Veröffentlichungstag und Bekanntmachung des Hinweises auf die Patenterteilung: **30.10.2024**
(21) Anmeldenummer: 21195963.0
(22) Anmeldetag: 10.09.2021
(51) Int. Cl.: G01N 21/77

(54) **OPTISCHER SENSOR, SYSTEM UND VERFAHREN ZUM NACHWEIS PATHOGENER KEIME**
OPTICAL SENSOR, SYSTEM AND METHOD FOR DETECTING PATHOGENS
CAPTEUR OPTIQUE, SYSTÈME ET PROCÉDÉ DE DÉTECTION DES GERMES PATHOGÈNES

(30) Priorität: 11.09.2020 DE 102020123800
(43) Veröffentlichungstag der Anmeldung: 16.03.2022
(73) Patentinhaber: Fraunhofer-Gesellschaft zur Förderung der angewandten Forschung e.V., 80686 München (DE)
(72) Erfinder: Schade, Wolfgang, 38640 Goslar (DE); Hübner, Eike, 38640 Goslar (DE); Reimer, Vladislav, 38640 Goslar (DE)
(74) Vertreter: Friese Goeden Patentanwälte PartGmbB

(56) Entgegenhaltungen:
- EP-B1- 1 509 593
- DE-B4- 102004 015 906
- US-A1- 2018 017 495

## Beschreibung

Die Erfindung betrifft einen optischen Sensor mit einem Substrat, welches zumindest eine erste Seite und eine gegenüberliegende zweite Seite aufweist, wobei zumindest auf der ersten Seite zumindest ein erster Wellenleiter angeordnet ist. Weiterhin betrifft die Erfindung ein Verfahren zum Nachweis pathogener Keime, bei welchen auf einen vorgenannten Sensor eine flüssige Probe aufgebracht und ein optisches Signal in das erste Ende des Wellenleiters eingekoppelt wird. Vorrichtungen und Verfahren dieser Art können zum Nachweis von pathogenen Keimen verwendet werden, welche in anderen Organismen gesundheitsschädigende Abläufe verursachen. Als pathogene Keime werden nachfolgend Mikroorganismen oder subzellulären Erreger bezeichnet, beispielsweise Algen, Bakterien, Parasiten, Pilze oder Viren. Ein solches Verfahren ist z.B. aus US2018/0017495 A1 bekannt.

Aus Sarkaleh AK, Lahijani BV, Saberkari H, Esmaeeli A.: "Optical Ring Resonators: A Platform for Biological Sensing Applications". J Med Signals Sens. 2017, 7(3), 185-191 ist bekannt, einen optischen Ringresonator zum Nachweis von Biopartikeln einzusetzen. Durch Ablagerung einer polymeren Empfängerschicht des Zielpartikels an der Peripherie des optischen Ringresonators ist es möglich, die Existenz von Molekülen durch Berechnung der Verschiebung der spektralen Antwort des Ringresonators zu identifizieren. Dieser bekannte Sensor weist jedoch den Nachteil auf, dass die Auslese des Sensors einen hohen apparativen Aufwand erfordert. Die Anwendung dieses bekannten Verfahrens ist daher auf wenige, gut ausgestattete Labore beschränkt. Ausgehend vom Stand der Technik liegt der Erfindung daher die Aufgabe zugrunde, eine Vorrichtung und ein Verfahren zum Nachweis pathogener Keime anzugeben, welches einfach und schnell anzuwenden sind.

Die Aufgabe wird erfindungsgemäß durch eine Vorrichtung gemäß Anspruch 1 und ein Verfahren nach Anspruch 9 gelöst. Vorteilhafte Weiterbildungen der Erfindung finden sich in den abhängigen Ansprüchen.

Erfindungsgemäß wird ein optischer Sensor vorgeschlagen, welcher ein Substrat enthält. Das Substrat kann beispielsweise ein Polymer sein bzw. ein Polymer enthalten, beispielsweise Polycarbonat oder Cycloolefin-Copolymere (COC). Alternativ kann das Substrat auch Glas, Quarz oder ein Halbleitermaterial enthalten oder daraus bestehen. Ein Halbleitermaterial kann ausgewählt sein aus Germanium, Silizium, einem III-V-Verbindungshalbleiter oder Diamant. Das Substrat kann einen mehrschichtigen Aufbau aufweisen, beispielsweise eine erste Polymerschicht auf einem Glas, einem zweiten Polymer oder einem Halbleiter. Das Substrat kann eine Dicke von etwa 50 µm bis etwa 250 µm oder von etwa 80 µm bis etwa 150 µm oder von etwa 750 µm bis etwa 1250 µm aufweisen. Das Substrat kann eine polygonale oder runde Grundform aufweisen. Insbesondere kann das Substrat rechteckig sein und eine Länge und eine Breit von jeweils etwa 1 cm bis etwa 5 cm oder von etwa 0,5 cm bis etwa 3 cm aufweisen. Das Substrat weist eine erste Seite und eine gegenüberliegende zweite Seite auf, wobei die erste und die zweite Seite jeweils die größten Flächen des Substrates sind. Die erste Seite und die gegenüberliegende zweite Seite sind durch Seitenflächen miteinander verbunden.

Zumindest auf der ersten Seite des Substrates ist zumindest ein erster Wellenleiter angeordnet. Der Wellenleiter kann beispielsweise durch UV-Lithografie erzeugt werden. In einigen Ausführungsformen der Erfindung ist der Wellenleiter dadurch erhältlich, dass das Substrat zumindest teilweise beschichtet wird, beispielsweise in einem Sol-Gel-Verfahren, einer Vakuumdeposition oder einem Spin-Coating-Verfahren. Die solchermaßen hergestellte Schicht kann einen anderen Brechungsindex aufweisen als das Substrat und nachfolgend durch Maskieren mit einem Photolack, Belichten, Entwickeln und Ätzen teilweise entfernt werden, so dass ein Wellenleiter auf der Oberfläche des Substrates ensteht. In anderen Ausführungsformen der Erfindung kann ein homogenes Substrat durch Maskieren mit einem Photolack, Belichten, Entwickeln und Ätzen teilweise entfernt werden, so dass ein Wellenleiter auf der zweiten Seite des Substrates freigestellt wird.

Der Wellenleiter kann in eingen Ausführungsformen eine Höhe von etwa 1 µm bis etwa 50 µm oder von etwa 2 µm bis etwa 6 µm oder von etwa 35 µm bis etwa 45 µm und/oder eine Breite von etwa 1 µm bis etwa 1000 µm oder von etwa 10 µm bis etwa 125 µm oder von etwa 10 µm bis etwa 50 µm oder von etwa 80 µm bis etwa 120 µm oder von etwa 1 µm bis etwa 10 µm oder von etwa 500 µm bis etwa 1000 µm aufweisen.

Der Wellenleiter kann ein erstes Ende und ein gegenüberliegendes zweites Ende aufweisen. Sowohl das erste als auch das zweite Ende des Wellenleiters können an einer Kante des Substrats angeordnet sein. In anderen Ausführungsformen der Erfindung kann das erste Ende und das zweite Ende des Wellenleiters an zwei unterschiedlichen Kanten des Substrates angeordnet werden.

Der Wellenleiter enthält weiterhin zumindest eine erste Messstelle. An der Messstelle weist der Wellenleiter zumindest eine Unterbrechung auf. In anderen Ausführungsformen der Erfindung kann die Anzahl der Unterbrechungen des Wellenleiters größer sein und beispielsweise zwischen etwa 3 und etwa 60 Unterbrechungen oder zwischen etwa 10 und etwa 30 Unterbrechungen oder zwischen etwa 5 und etwa 20 Unterbrechungen oder zwischen etwa 50 und etwa 100 Unterbrechungen betragen. Zwischen zwei benachbarten Unterbrechungen des Wellenleiters befindet sich wiederum ein vergleichsweise kurzes Stück des Wellenleiters, dessen Länge in etwa der Länge der Unterbrechung entspricht. In anderen Ausführungsformen der Erfindung kann der Wellenleiter zwischen zwei Unterbrechungen zwischen etwa einem Faktor 2 und etwa einem Faktor 20 oder zwischen einem Faktor 1 und einem Faktor 10 größer sein als die Unterbrechung. Unter einer Unterbrechung des Wellenleiters wird insofern für die Zwecke der vorliegenden Beschreibung eine oder mehrere Lücken im Verlauf des Wellenleiters bezeichnet, welche von einem optischen Signal sequentiell durchlaufen werden.

Bei Betrieb des optischen Sensors wird am ersten Ende des Wellenleiters ein optisches Signal eingekoppelt und am zweiten Ende des Wellenleiters mittels eines Detektors nachgewiesen. An den Unterbrechungen wird ein Teil des eingekoppelten Lichtes reflektiert, so dass die im Detektor nachgewiesene transmittierte Intensität geringer ist als die eingekoppelte Intensität. Befindet sich auf der Messstelle ein flüssiges, insbesondere wässriges Medium, verringert sich der Brechzahlunterschied zwischen dem Material des Wellenleiters und dem flüssigen Medium, so dass die durch den Wellenleiter transmittierte Leistung ansteigt. Befinden sich im flüssigen Medium pathogene Keime, so wirken diese Partikel als Streuzentren, welche einen Teil des eingekoppelten Lichtes aus dem Wellenleiter herausstreuen. Dadurch nimmt die im Detektor nachgewiesene Intensität wieder ab, so dass die Anwesenheit der Partikel und deren Anzahl nachgewiesen werden kann.

In einigen Ausführungsformen der Erfindung wird das in den ersten Wellenleiter eingekoppelte optische Signal so ausgewählt, dass zumindest ein Teil des Spektrums eine Wellenlänge aufweist, welche größer oder mindestens gleich dem Durchmesser der nachzuweisenden Partikel ist. In einigen Ausführungsformen der Erfindung kann die Wellenlänge um etwa einen Faktor 4 bis etwa einen Faktor 7 größer sein als die nachzuweisenden Partikel. Die Lichtquelle kann in einigen Ausführungsformen der Erfindung eine LED, eine Superlumineszenzdiode oder ein Halbleiterlaser sein oder enthalten. Das optische Signal kann in einigen Ausführungsformen der Erfindung monochromatisch sein. In anderen Ausführungsformen der Erfindung kann das optische Signal eine Mehrzahl unterschiedlicher Wellenlängen bzw. mehrere Wellenlängenbereiche aufweisen oder eine Weißlichtquelle sein.

Der mit dem zweiten Ende des ersten Wellenleiters gekoppelte Detektor kann eine an sich bekannte Photodiode oder ein Photodiodenarray sein. In einigen Ausführungsformen der Erfindung kann der Detektor ein Spektrometer enthalten, insbesondere ein mikrooptisches Spektrometer oder ein Arrayed-Waveguide-Grating (AWG). Solche Elemente können optional auf dem Substrat integriert sein. In einigen Ausführungsformen der Erfindung kann der Detektor ein CCD- oder ein CMOS-Detektor sein, welcher als Zeilen- oder Flächendetektor ausgebildet sein kann. Ein solcher Zeilen- oder Flächendetektor kann eine Mehrzahl von Pixeln enthalten und dadurch eine räumliche Auflösung gestatten. Einzelne Pixel können in Subpixel unterteilt sein und auf diese Weise eine Detektion verschiedener Wellenlängen bzw. verschiedener Wellenlängenbereiche ermöglichen. Ein solcher Detektor kann somit ein an sich bekannter Kamerachip sein.

In einigen Ausführungsformen der Erfindung können die Unterbrechungen des ersten Wellenleiters in der ersten Messstelle periodisch angeordnet sein. In einigen Ausführungsformen der Erfindung können die Unterbrechungen des ersten Wellenleiters in der ersten Messstelle eine Länge von etwa 2 µm bis etwa 20 µm oder eine Länge von etwa 3 µm bis etwa 10 µm oder eine Länge von etwa 2 µm bis etwa 6 µm aufweisen. Die genannten Abmessungen ermöglichen eine zuverlässige Anlagerung der nachzuweisenden Partikel bzw. pathogenen Keime und ein günstiges Signal-/Rauschverhältnis.

In einigen Ausführungsformen der Erfindung kann am ersten Ende des Wellenleiters und/oder am gegenüberliegenden zweiten Ende des Wellenleiters jeweils ein Koppler angeordnet sein. Ein solcher Koppler kann in einigen Ausführungsformen ausgewählt sein aus einem Gitterkoppler oder einem Lichttrichter bzw. Taper. Hierdurch kann die Effizienz der Ein- bzw. Auskopplung zwischen Lichtquelle und Detektor erhöht werden, so dass die Nachweisgrenze und/oder die Messgenauigkeit verbessert sein können.

Erfindungsgemäß ist die erste Messstelle durch zumindest eine Beschichtung funktionalisiert. Eine solche Funktionalisierung erlaubt die selektive Ankopplung vorgebbarer Mikroorganismen oder subzellulärer Erreger, so dass ein spezifischer Nachweis bestimmter pathogener Keime ermöglicht wird, ohne dass andere Keime und/oder anorganische Partikel die Messgenauigkeit beeinflussen.

Die Beschichtung kann in einigen Ausführungsformen ein Mehrschichtsystem enthalten bzw. daraus bestehen, welches eine erste Schicht umfasst, welche Al₂O₃ enthält und eine Schichtdicke von etwa 10 nm bis etwa 50 nm aufweist. Die Herstellung dieser Schicht kann in einigen Ausführungsformen der Erfindung dadurch erfolgen, dass eine Aluminiumbeschichtung aufgebracht wird, beispielsweise durch ein Sputterverfahren oder durch thermisches Aufdampfen, welche nachfolgend unter Zugabe von Sauerstoff einem Plasma ausgesetzt wird, so dass die Aluminiumschicht zu Al₂O₃ oxidiert wird.

Auf diese Schicht kann in einigen Ausführungsformen der Erfindung ein Silan angebunden werden, welches Bindungsstellen für biologische Moleküle anbietet. In einigen Ausführungsformen der Erfindung kann (3-Glycidyloxypropyl)-trimethoxysilan an die terminale Al-OH-Gruppe der Al₂O₃-schicht aufgebracht werden. Im letzten Schritt der Funktionalisierung können Antikörper zur selektiven Anbindung der nachzuweisenden Viren über eine Reaktion ihrer Aminogruppen mit der Epoxid-Funktionalität des immobilisierten Silans aufgebracht werden. Die an die Antikörper gebundenen Viren wirken sodann als Streuzentren auf den Flächen der ersten Messstelle des ersten Wellenleiters. Erfindungsgemäß enthält der Sensor weiterhin einen zweiten Wellenleiter, welcher zumindest eine zweite Messstelle aufweist, welche zumindest eine Unterbrechung des zweiten Wellenleiters enthält. Erfindungsgemäß ist die zweite Messstelle nicht durch zumindest eine Beschichtung funktionalisiert, wie diese vorstehend im Zusammenhang mit der ersten Messstelle beschrieben wurde. Der geometrische Aufbau der ersten und der zweiten messstelle kann in einigen Ausführungsformen der Erfindung identisch sein. Auf diese Weise kann mit dem zweiten Wellenleiter ein Referenzsignal erzeugt werden, so dass durch differenzielle Messung der aus den Wellenleitern austretenden Lichtintensität die Genauigkeit des Messverfahrens erhöht sein kann.

In einigen Ausführungsformen der Erfindung kann der Sensor weiterhin einen dritten Wellenleiter enthalten, welcher ein erstes Ende und ein gegenüberliegendes zweites Ende auf-weist. Das zweite Ende kann benachbart zum zweiten Ende des ersten und/oder zweiten Wellenleiters im Substrat angeordnet sein, so dass die im dritten Wellenleiter geführten optischen Signale ebenfalls mit einem optischen Detektor oder einer Teilfläche eines ortsauflösenden Detektors nachweisbar sind. Das erste Ende des dritten Wellenleiters kann an die erste Messstelle herangeführt sein, so dass Streulicht aus der ersten Messstelle in den dritten Wellenleiter einkoppelt. Die Anwesenheit der nachzuweisenden Partikel zeigt sich somit in einer Schwächung des optischen Signals im ersten Wellenleiter und einem Anstieg des optischen Signals im dritten Wellenleiter.

In einigen Ausführungsformen der Erfindung kann der Sensor weiterhin einen vierten Wellenleiter enthalten, welcher ein erstes Ende und ein gegenüberliegendes zweites Ende auf-weist. Das zweite Ende kann benachbart zum zweiten Ende des ersten und/oder zweiten und/oder dritten Wellenleiters im Substrat angeordnet sein, so dass die im vierten Wellenleiter geführten optischen Signale ebenfalls mit einem optischen Detektor oder einer Teilfläche eines ortsauflösenden Detektors nachweisbar sind. Das erste Ende des vierten Wellenleiters kann an die zweite Messstelle herangeführt sein, so dass Streulicht aus der zweiten Messstelle in den vierten Wellenleiter einkoppelt.

Der erfindungsgemäß vorgeschlagene Sensor und das erfindungsgemäß vorgeschlagene Verfahren eignen sich insbesondere zum Nachweis pathogener Keime in flüssigen Proben. In einigen Ausführungsformen der Erfindung kann das Aufbringen der flüssigen Probe auf die erste und/oder zweite Messstelle folgende Schritte umfassen: Aufbringen der flüssigen Probe auf die Messstelle, Abwarten einer vorgebbaren Zeitspanne, Abwaschen der flüssigen Probe mit einem Lösungsmittel und Aufbringen einer Referenzflüssigkeit zumindest auf die erste Messstelle und/oder auf die zweite Messstelle. Die vorgebbare Zeitspanne kann in einigen Ausführungsformen der Erfindung zwischen etwa 1 Minute und etwa 10 Minuten oder zwischen etwa 3 Minuten und etwa 5 Minuten betragen. Das zum Abwaschen der flüssigen Probe verwendete Lösungsmittel kann in einigen Ausführungsformen der Erfindung destilliertes Wasser oder isotonische Kochsalzlösung oder eine Phosphat gepufferte Salzlösung (PBS) sein. Eine PBS hält nicht nur den Salzgehalt, sondern auch den pH-Wert innerhalb vorgebbarer Grenzwerte. Sofern die flüssige Probe eine fettige Phase enthält, können auch Kohlenwasserstoffe zum Abwaschen der flüssigen Probe verwendet werden. Die Referenzflüssigkeit kann in einigen Ausführungsformen der Erfindung mit dem zum Abwaschen der flüssigen Probe verwendeten Lösungsmittel identisch sein und beispielsweise isotonische Kochsalzlösung, eine andere Salzlösung, eine Phosphat gepufferte Salzlösung (PBS) oder destilliertes Wasser enthalten oder daraus bestehen. Die flüssige Probe kann ausgewählt sein aus Speichel, Blut, Liquor, Schweiß, Urin oder anderen Körperflüssigkeiten, welche nachzuweisende pathogene Keime enthalten können.

Während dem Einwirken der flüssigen Probe auf die Messstellen können pathogene Keime an den Antikörpern der funktionalisierten Oberflächen gebunden werden, wie vorstehend beschrieben wurde. Beim Abwaschen der flüssigen Probe mit dem Lösungsmittel wird überschüssiges Probenmaterial entfernt. Die an der funktionalisierten Oberfläche haftenden pathogenen Keime verbleiben jedoch in der ersten Messstelle. Das Aufbringen einer Referenzflüssigkeit mit vorgebbarem Brechungsindex dient dazu, das Licht verlustarm durch die Unterbrechungen der Messstellen zu leiten, so dass aus dem Unterschied der gemessenen Intensitäten an den zweiten Ende der Wellenleiter der Anteil gestreuten Lichtes und damit die Konzentration der pathogenen Keime an der ersten Messstelle bestimmt werden kann.

In einigen Ausführungsformen der Erfindung kann eine einzige Lichtquelle vorhanden sein, welche das optische Signal erzeugt, welches sowohl in den ersten als auch in den zweiten Wellenleiter eingekoppelt wird. Hierdurch werden Intensitätsschwankungen aufgrund unterschiedlicher Eigenschaften verschiedener Lichtquellen ausgeschlossen, so dass die Lichtintensität in beiden Wellenleitern identisch ist. Dies erlaubt es, ein zuverlässiges Referenzsignal zu erhalten.

In einigen Ausführungsformen der Erfindung kann der erfindungsgemäße Sensor mit einem Mobiltelefon ausgelesen werden. Eine im Mobiltelefon angeordnete Leuchtdiode, Superlumineszenzdiode oder auch Laserdiode kann als Lichtquelle dienen und die im Mobiltelefon vorhandene Kamera als Detektor. Zur mechanischen Befestigung des erfindungsgemäßen Sensors am Mobiltelefon und zur reproduzierbaren Positionierung der ersten und zweiten Enden der Wellenleiter vor der Lichtquelle und der Kamera kann das Mobiltelefon mit einem Aufsatz versehen werden, welcher formschlüssig am Mobiltelefon befestigt ist und seinerseits den Sensor formschlüssig aufnimmt.

Nachfolgend soll die Erfindung anhand von Figuren ohne Beschränkung des allgemeinen Erfindungsgedankens näher erläutert werden. Dabei zeigt
Fig. 1 ein Ausführungsbeispiel eines erfindungsgemäßen Sensors in der Aufsicht.
Fig. 2 zeigt eine vergrößerte Darstellung der ersten Messstelle in der Aufsicht.
Fig. 3 zeigt eine vergrößerte Darstellung der ersten Messstelle im Schnitt.
Fig. 4 zeigt eine vergrößerte Darstellung aus Fig. 3.
Fig. 5 zeigt ein Mobiltelefon mit einem erfindungsgemäßen Sensor.
Fig. 6 zeigt ein Referenzsignal eines erfindungsgemäßen Sensors.
Fig. 7 zeigt ein Messsignal eines erfindungsgemäßen Sensors.

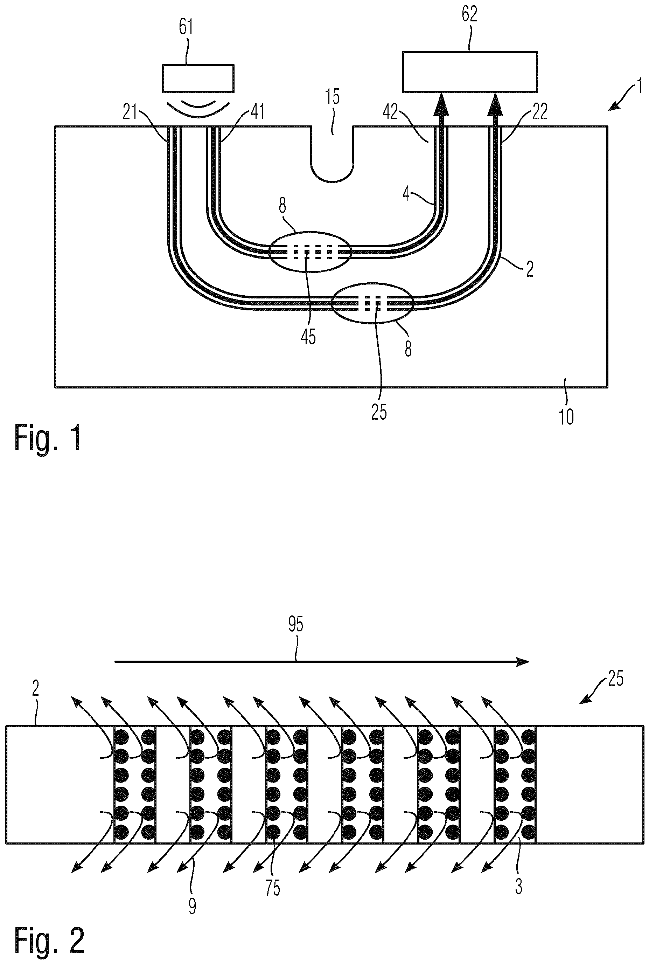

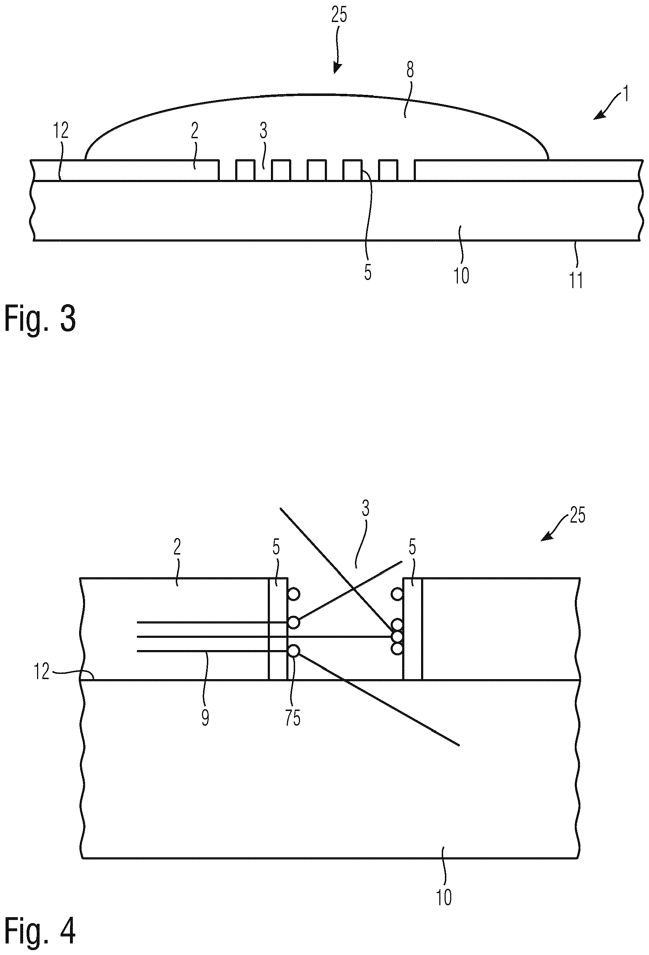

Anhand der Fig. 1, 2, 3 und 4 wird ein erfindungsgemäßer Sensor näher erläutert. Dabei zeigt Fig. 1 eine Aufsicht auf den Sensor, Fig. 2 zeigt eine vergrößerte Darstellung der ersten Messstelle 25 in der Aufsicht, Fig. 3 zeigt die erste Messstelle 25 im Schnitt und Fig. 4 zeigt einen vergrößerten Ausschnitt aus Fig. 3.

Der Sensor 1 umfasst ein Substrat 10, welches im dargestellten Ausführungsbeispiel eine rechteckige Grundform mit einer Länge und einer Breite von jeweils etwa 0,5 cm bis etwa 3 cm aufweist. Das Substrat weist eine Kerbe 15 auf, um dieses beim Einsetzen in ein Auslesegerät oder ein Gehäuse reproduzierbar zu positionieren. Durch photolithografische Verfahren, beispielsweise UV-Lithografie, sind auf dem Substrat 10 ein erster Wellenleiter 2 und ein zweiter Wellenleiter 4 ausgebildet. Der erste Wellenleiter 2 enthält eine erste Messstelle 25. Der zweite Wellenleiter 4 enthält eine zweite Messstelle 45. Der erste Wellenleiter 2 weist ein erstes Ende 21 auf. Der zweite Wellenleiter 4 weist ein erstes Ende 41 auf. Beide Wellenleiter weisen ein gegenüberliegendes zweites Ende 22 bzw. 42 auf.

Für den Betrieb des Sensors werden die ersten Enden mit einer Lichtquelle 61 gekoppelt, welche im dargestellten Ausführungsbeispiel eine Weißlichtquelle ist, beispielsweise eine Superlumineszenzdiode. In anderen Ausführungsbeispielen kann die Lichtquelle auch monochromatisches Licht aussenden und beispielsweise einen Halbleiterlaser umfassen. Die ersten Enden 21 und 41 des ersten und zweiten Wellenleiters 2 und 4 sind so eng innerhalb des Substrates 10 geführt, dass das Licht einer einzigen Lichtquelle 61 in beide Wellenleiter eingekoppelt werden kann. Hierdurch kann die in beide Wellenleiter eingekoppelte Intensität identisch sein und zeitliche Schwankungen der Lichtintensität betreffen beide Wellenleiter.

Die zweiten Enden 22 und 42 der Wellenleiter 2 und 4 sind mit einem Detektor 62 gekoppelt. Der Detektor 62 kann beispielsweise ein zweidimensionaler CCD-Chip sein. Dieser enthält eine Mehrzahl von Pixeln, welche eine ortsaufgelöste Erfassung eintreffenden Lichtes ermöglichen. Hierdurch kann am Detektor 62 unterschieden werden, welche Lichtintensität aus jedem der Wellenleiter 4 und 2 den Detektor 62 erreicht. In anderen Ausführungsformen der Erfindung kann der Detektor 62 auch eine Photodiode oder ein Photodiodenarray oder ein anderer, an sich bekannter photoelektrischer Wandler sein. Das aus dem zweiten Wellenleiter 4 eintreffende Licht kann somit als Referenzsignal verwendet werden und das aus dem ersten Wellenleiter 2 eintreffende Licht kann als Messsignal verwendet werden. Durch differentielle Auswertung des Messsignales kann somit die Messgenauigkeit erhöht sein.

Weiterhin kann jedes Pixel des Detektors 62 eine Mehrzahl von Subpixeln enthalten, so dass der Detektor 62 auf unterschiedliche Wellenlängen bzw. Wellenlängenbereiche selektiv ist. Beispielsweise kann der Detektor 62 die Intensität von rotem, grünem und blauem Licht getrennt voneinander erfassen.

Wie aus Fig. 3 und 4 ersichtlich ist, weist das Substrat 10 eine erste Seite 11 und eine gegenüberliegende zweite Seite 12 auf. Auf der zweiten Seite 12 wurden die Wellenleiter 2 und 4 durch Aufbringen einer flüssigen Schicht mittels spin-coating, UV-lithographischer Härtung der Wellenleiterstruktur und Abwaschen der nicht ausgehärteten Bereiche hergestellt. Hierdurch ist der Wellenleiter 2 auf der Oberfläche des Substrates 10 so hergestellt, dass dieser als Rippenwellenleiter auf der zweiten Seite 12 des Substrates 10 erhaben ausgeformt wird. Beispielsweise kann der Wellenleiter 2 eine Breite von etwa 100 µm und eine Höhe von etwa 5 µm aufweisen. Die Messstelle 25 des Wellenleiters 2 enthält eine Mehrzahl von Unterbrechungen 3. Die Unterbrechungen 3 können beispielsweise eine in Längsrichtung 95 des Wellenleiters gemessene Länge von etwa 3 µm bis etwa 5 µm aufweisen. Die Unterbrechungen 3 des Wellenleiters 2 werden bei Betrieb des Sensors von einem optischen Signal sequentiell durchlaufen.

Das Material der beiden Wellenleiter 2 und 4 kann einen Brechungsindex zwischen etwa 1,4 und etwa 1,8 oder zwischen etwa 1,4 und etwa 1,6 aufweisen. Hierzu können die Wellenleiter 2 und 4 und/oder das Substrat 10 beispielsweise aus einem Glas oder einem Kunststoff bestehen, beispielsweise Polycarbonat oder Epoxidharz.

In Fig. 1 und Fig. 3 ist weiterhin dargestellt, dass auf die Messstelle 25 eine Referenzflüssigkeit 8 aufgebracht ist, welche die Unterbrechungen 3 ausfüllt. Die Referenzflüssigkeit 8 kann beispielsweise destilliertes Wasser sein und einen Brechungsindex von 1,3 aufweisen. Die in die Unterbrechungen 3 eindringende Flüssigkeit reduziert den Brechzahlunterschied zwischen dem Wellenleiter 2 und der Unterbrechung 3, so dass am Detektor 62 eine höhere Lichtintensität gemessen wird, wenn die Flüssigkeit 2 auf der Messstelle 25 vorhanden ist. Gleiches gilt für die zweite Messstelle 45 im zweiten Wellenleiter 4.

Fig. 4 zeigt einen vergrößerten Ausschnitt der Messstelle 25. Dargestellt ist eine einzelne Unterbrechung 3 aus der Mehrzahl von Unterbrechungen, welche an der Messstelle 25 vorhanden sind.

Wie in Fig. 4 erkennbar ist, sind zumindest die Stirnseiten des Wellenleiters 2, welche an die Unterbrechung angrenzen, mit einer Beschichtung 5 versehen. In einigen Ausführungsformen der Erfindung kann die Oberfläche des Wellenleiters 2 auch vollständig beschichtet sein. Die Beschichtung 5 bewirkt eine Funktionalisierung der Oberfläche dahingehend, dass die nachzuweisenden pathogenen Keime, beispielsweise Viren, an die Oberfläche gebunden werden. Andere Partikel, beispielsweise anorganische Partikel oder andere Keime, welche nicht nachgewiesen werden sollen, haften hingegen nicht an der Beschichtung 5. In einigen Ausführungsformen der Erfindung kann die Beschichtung 5 hierzu ein Mehrschichtsystem enthalten, welches selektive Antikörper haftfest an die Oberfläche des Wellenleiters 2 zumindest in der Unterbrechung 3 bindet.

Die zweite Messstelle 45 im zweiten Wellenleiter 4 ist grundsätzlich ähnlich oder auch identisch aufgebaut wie die erste Messstelle 25, enthält jedoch keine Beschichtung 5 oder zumindest keine Funktionalisierung.

Zur Durchführung einer Messung wird eine flüssige Probe sowohl auf die erste Messstelle 25 als auch auf die zweite Messstelle 45 aufgebracht. Dabei kann es sich beispielsweise um Speichel, Liquor, Blut, jede andere Körperflüssigkeit oder auch eine von einer Oberfläche eines unbelebten Objekts gewonnene Probe handeln. Nach einer vorgebbaren Zeitspanne, welche zwischen etwa 1 Minute und etwa 5 Minuten betragen kann, werden beide Messstellen 25 und 45 mit einem Lösungsmittel gereinigt. Das Lösungsmittel kann beispielsweise destilliertes Wasser, eine gepufferte Salzlösung oder ein anderes, hier nicht explizit genanntes Lösungsmittel enthalten. Das Lösungsmittel ist dazu eingerichtet und bestimmt, die Reste der flüssigen Probe von den Messstellen 25 und 45 abzuwaschen.

Sofern pathogene Keime 75 in der flüssigen Probe enthalten waren, haften diese an den Antikörpern der Beschichtung 5 der ersten Messstelle 25. Da die zweite Messstelle 45 nicht funktionalisiert ist, haften die pathogenen Keime dort nicht. Vielmehr werden diese mit dem Lösungsmittel abgewaschen.

Nachfolgend wird eine Referenzflüssigkeit 8 auf beide Messstellen aufgebracht, welche beispielsweise Wasser oder eine wässrige Lösung enthalten kann.

Schließlich wird ein optisches Signal in den ersten Wellenleiter 2 und den zweiten Wellenleiter 4 eingekoppelt. Das optische Signal im zweiten Wellenleiter 4 wird durch die Unterbrechungen 3 der zweiten Messstelle 45 geschwächt und erreicht als Referenzsignal den Detektor 62. Das optische Signal im ersten Wellenleiter 2 unterliegt der identischen Schwächung. Wie aus Figur 2 und Figur 4 erkennbar ist, wird das optische Signal 9 zusätzlich an den auf der Beschichtung 5 anhaftenden pathogenen Keimen gestreut. Das gestreute Licht verlässt den Wellenleiter 2, so dass im Wellenleiter 2 bei Anwesenheit pathogener Keine eine zusätzliche Schwächung des optischen Signals im Detektor 62 auftritt. Auf diese Weise können beispielsweise Viren mit einem typischen Durchmesser von etwa 100 nm bis etwa 150 nm und einer typischen Brechzahl von etwa 1,4 bis etwa 1,6 nachgewiesen werden. Durch Vergleich des Referenzsignals aus dem zweiten Wellenleiter 4 und dem Messsignal aus dem ersten Wellenleiter 2 kann somit die Anwesenheit und die Konzentration der pathogenen Keime 75 nachgewiesen werden.

Der erfindungsgemäße Sensor 1 zeichnet sich dadurch aus, dass die Auslese mit geringem apparativem Aufwand möglich ist. Beispielsweise kenn ein Mobiltelefon oder ein Tablet-Computer mit eingebauter Lichtquelle und eingebauter Kamera zur Signalerfassung und Signalverarbeitung verwendet werden.

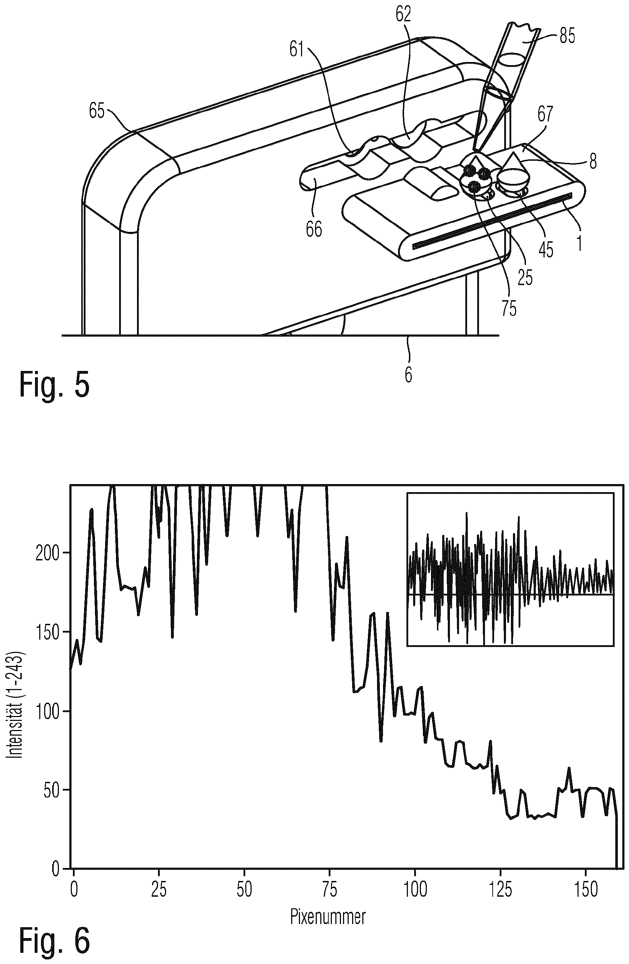

Wie in Fig. 5 dargestellt ist, kann ein Mobiltelefon 6 mit einem Passstück 65 versehen werden. Das Passstück 65 kann formschlüssig auf das Mobiltelefon 6 aufgesteckt werden und dort durch Klemmung oder Klebung befestigt sein. Das Passstück 65 enthält eine Aussparung 66, welche komplementär zu einem Gehäuse 67 des Sensors 1 geformt ist. Weiterhin ist die Aussparung 66 so geformt, dass die zweiten Enden 22 und 42 der Wellenleiter 2 und 4 der Kameralinse gegenüberstehen und die Kamera dadurch als Detektor 62 verwendbar ist. Weiterhin ist die Aussparung 66 so geformt, dass die ersten Enden 21 und 41 der Wellenleiter 2 und 4 der im Mobiltelefon ohnehin vorhandene Lichtquelle 61 gegenüberstehen, so dass das Mobiltelefon auch zur Erzeugung des optischen Signals 9 verwendbar ist.

Wie aus Fig. 5 erkennbar ist, ist der Sensor 1 in ein Gehäuse 67 eingesetzt, welches zwei Aussparungen aufweist, so dass die erste Messstelle 25 und die zweite Messstelle 45 zur Umgebung durch zugeordnete Bohrungen im Gehäuse exponiert sind. Weiterhin weist das Gehäuse 67 Koppelelemente auf, welche die Positionierung des ersten Endes 21 des ersten Wellenleiters 2 und des ersten Ende 41 des zweiten Wellenleiters 4 vor der Lichtquelle 61 erlauben. In gleicher Weise sind die zweiten Enden 22 und 42 der Wellenleiter 2 und 4 vor der Kameralinse des Mobiltelefons 6 positioniert, welche als Detektor 62 verwendet wird. Durch die Form der Ausnehmung 66 und der komplementären Form des Gehäuses 67 kann ein falsches Einsetzen des Gehäuses 67 durch Formschluss verhindert werden.

Zur Durchführung der Messung wird die flüssige Probe mittels einer Pipette 85 auf die beiden Messstellen 25 und 45 aufgebracht. Nach ausreichender Kontaktzeit der pathogenen Keime 75 mit der Beschichtung 5 können die Messstellen 25 und 45 abgewaschen und die Messung durchgeführt werden, wie vorstehend beschrieben. Das zur Auslese verwendete Mobiltelefon kann die Steuerung des Messverlaufes, die Signalauslese und die Speicherung der Messdaten und ggf. auch die Übertragung der Messdaten auf einen Zentralcomputer übernehmen. Hierzu kann auf dem Mobiltelefon 6 ein entsprechendes Computerprogramm ausgeführt werden, welches zumindest Teilschritte des erfindungsgemäßen Messverfahrens durchführt, während das Programm auf dem Mikroprozessor des Mobiltelefons 6 läuft. Der Sensor 1 mit dem Gehäuse 67 kann als Einwegprodukt nach Durchführung der Messung entsorgt werden.

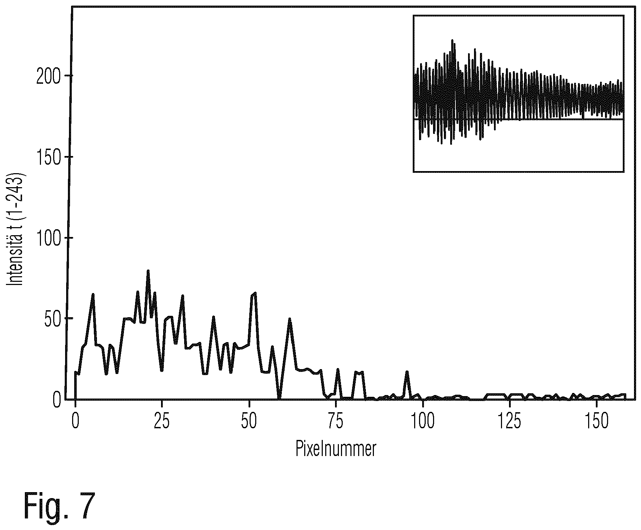

Anhand der Fig. 6 und 7 wird nochmals die Durchführung einer erfindungsgemäßen Messung erläutert. Dabei zeigt Fig. 6 das aus dem zweiten Wellenleiter 4 gewonnene Referenzsignal und Fig. 7 das im ersten Wellenleiter 2 erzeugte Messsignal.

Die Lichtquelle 61 erzeugt Laserlicht mit einer Wellenlänge von 650 nm. Der verwendete Detektor 62 erfasst Licht in drei Wellenlängenbereichen. Im dargestellten Ausführungsbeispiel wird jedoch nur ein Teilspektrum aus dem roten Spektralbereich entsprechend der Wellenlänge des Laserlichtes zur Messung verwendet. Dargestellt ist in beiden Figuren jeweils die Intensität auf der Ordinate und der Ort auf der Abszisse entlang einer Linie des zweidimensionalen Detektors. Da der Detektor mit 8 Bit Auflösung betrieben wird, ergeben sich für die Ordinate 2⁸=256 Stufen der Intensität. Eingeblendet ist zusätzlich das zweidimensionale Bild des Detektors, wobei die Intensität in Graustufen kodiert ist.

Wie aus Fig. 6 ersichtlich ist, wird durch den zweiten Wellenleiter 4 eine erhebliche Lichtintensität transmittiert, so dass die Intensität des Refrenzsignals in mehreren Pixeln die Sättigungsintensität erreicht.

Durch die Anwesenheit von Partikeln mit einem Durchmesser von 140 nm und einem Brechungsindex von 1,4 auf der funktionalisierten Schicht in den Unterbrechungen 3 der Messstelle 25 des ersten Wellenleiters 2 wird das rote Licht soweit gestreut, dass die Intensität um mehr als einen Faktor 5 abnimmt. Durch differentielle Messung bzw. Vergleich des in Fig. 6 gezeigten Referenzsignals mit dem in Fig. 7 gezeigten Messsignal lässt sich somit die Anwesenheit der Partikel eindeutig nachweisen und quantifizieren.

Es ist darauf hinzuweisen, dass der zweite Wellenleiter 4 zur Erzeugung des Referenzsignals optional ist und in einigen Ausführungsformen der Erfindung auch entfallen kann. In diesem Fall kann das Messsignal und das Referenzsignal auch mittels eines einzigen Wellenleiters sequenziell erfasst werden und/oder das Messsignal kann mittels Kalibrierdaten aus einer Datenbank ausgewertet werden. Selbstverständlich ist die Erfindung nicht auf die dargestellten Ausführungsformen beschränkt. Die vorstehende Beschreibung ist daher nicht als beschränkend, sondern als erläuternd anzusehen. Die nachfolgenden Ansprüche sind so zu verstehen, dass ein genanntes Merkmal in zumindest einer Ausführungsform der Erfindung vorhanden ist. Dies schließt die Anwesenheit weiterer Merkmale nicht aus. Sofern die Ansprüche und die vorstehende Beschreibung "erste" und "zweite" Ausführungsformen definieren, so dient diese Bezeichnung der Unterscheidung zweier gleichartiger Ausführungsformen, ohne eine Rangfolge festzulegen.

Die Erfindung ist durch die Ansprüche definiert.

## Patentansprüche

1. Optischer Sensor (1) mit einem Substrat (10), welches zumindest eine erste Seite (11) und eine gegenüberliegende zweite Seite (12) aufweist, wobei zumindest auf der ersten Seite (11) zumindest ein erster Wellenleiter (2) angeordnet ist, welcher zumindest eine erste Messstelle (25) aufweist, welche zumindest eine Unterbrechung (3) des ersten Wellenleiters (2) aufweist, **dadurch gekennzeichnet, dass**
die erste Mess¬stelle (25) durch zumindest eine Beschichtung (5) funktionalisiert ist, welche die selektive Ankopplung vorgebbarer Mikroorganismen oder subzellulärer Erreger erlaubt, wobei
der Sensor weiterhin zumindest einen zweiten Wellenleiter (4) enthält, welcher zumindest eine zweite Mess¬stelle (45) aufweist, welche zumindest eine Unter-brechung (3) des zweiten Wellenleiters (4) aufweist und nicht durch zumindest eine Beschichtung funktionalisiert ist.

2. Sensor nach Anspruch 1, **dadurch gekennzeichnet, dass** die erste Messstelle (25) zwischen etwa 3 und etwa 60 Unterbrechungen (3) aufweist oder dass die erste Messstelle (25) zwischen etwa 10 und etwa 30 Unterbrechungen (3) aufweist oder dass die erste Messstelle (25) zwischen etwa 5 und etwa 20 Unterbrechungen (3) aufweist oder dass die erste Messstelle (25) zwischen etwa 50 und etwa 100 Unterbrechungen (3) aufweist.

3. Sensor nach Anspruch 1 oder 2, **dadurch gekennzeichnet,**
**dass** die Unterbrechungen (3) des ersten Wellenleiters (2) in der ersten Messstelle (25) periodisch angeordnet sind oder
**dass** die Unterbrechungen (3) des ersten Wellenleiters (2) in der ersten Messstelle (25) eine Länge von etwa 2 µm bis etwa 20 µm oder
eine Länge von etwa 3 µm bis etwa 10 µm oder
eine Länge von etwa 2 µm bis etwa 6 µm aufweisen.

4. Sensor nach einem der Ansprüche 1 bis 3, **dadurch gekennzeichnet, dass** der Wellenleiter (2) ein erstes Ende (21) und ein gegenüberliegendes zweites Ende (22) aufweist, wobei sich am ersten Ende (21) und/oder am zweiten Ende (22) jeweils ein Koppler befindet.

5. Sensor nach einem der Ansprüche 1 bis 4, **dadurch gekennzeichnet, dass** das Substrat ein Glas oder ein Polymer oder einen Halbleiter enthält oder daraus besteht.

6. Sensor nach einem der Ansprüche 1 bis 5, weiterhin enthaltend zumindest einen dritten Wellenleiter, welcher ein erstes Ende und ein gegenüberliegendes zweites Ende auf-weist, wobei das zweite Ende dazu eingerichtet ist, mit einem Detektor (62) gekoppelt zu werden und das erste Ende des dritten Wellenleiters an die erste Messstelle (25) herangeführt ist, dass Streulicht aus der ersten Messstelle (25) in den dritten Wellenleiter einkoppelbar ist.

7. Sensor nach einem der Ansprüche 1 bis 6 weiterhin enthaltend zumindest einen vierten Wellenleiter, welcher ein erstes Ende und ein gegenüberliegendes zweites Ende auf-weist, wobei das zweite Ende dazu eingerichtet ist, mit einem Detektor (62) gekoppelt zu werden und das erste Ende des vierten Wellenleiters an die zweite Messstelle (45) herangeführt ist, dass Streulicht aus der zweiten Messstelle (45) in den vierten Wellenleiter einkoppelbar ist.

8. Sensor nach einem der Ansprüche 1 bis 7, weiterhin enthaltend zumindest eine Lichtquelle (61) und/oder zumindest einen Detektor (62), wobei die Lichtquelle (61) mit dem ersten Ende (21) des Wellenleiters (2) verbindbar ist und der Detektor (62) mit dem zweiten Ende (21) des Wellenleiters (2) verbindbar ist.

9. Verfahren zum Nachweis pathogener Keime mit folgenden Schritten:
Bereitstellen eines optischen Sensors (1) mit einem Substrat (10), welches zumindest eine erste Seite (11) und eine gegenüberliegende zweite Seite (12) aufweist, wobei zumindest auf der ersten Seite (11) zumindest ein erster Wellenleiter (2) angeordnet ist, welcher ein erstes Ende (21) und ein gegenüberliegendes zweites Ende (22) und zumindest eine erste Messstelle (25) aufweist, welche zumindest eine Unterbrechung (3) des ersten Wellenleiters (2) enthält und wobei die erste Mess¬stelle (25) durch zumindest eine Beschichtung (5) funktionalisiert ist, wobei die Beschichtung die selektive Ankopplung vorgebbarer Mikroorganismen oder subzellulärer Erreger erlaubt, und
der Sensor weiterhin zumindest einen zweiten Wellenleiter (4) enthält, welcher ein erstes Ende (21) und ein gegenüberliegendes zweites Ende (22) und zumindest eine zweite Mess¬stelle (45) aufweist, welche zumindest eine Unterbrechung (3) des zweiten Wellenleiters (4) auf-weist und nicht durch zumindest eine Beschichtung funktionalisiert ist;
Aufbringen einer flüssigen Probe, welche pathogene Keime (75) enthält, zumindest auf die erste Messstelle (25) ;
Einkoppeln eines optischen Signals (9) in das erste Ende (21) des ersten Wellenleiters (2);
Bestimmen der Intensität des optischen Signals (9) am zweiten Ende (22) des ersten Wellenleiters (2);

10. Verfahren nach Anspruch 9, **dadurch gekennzeichnet, dass** das optische Signal (9) eine einzige Wellenlänge aufweist und die Intensität des optischen Signals (9) mit dieser Wellenlänge am zweiten Ende (22) des Wellenleiters bestimmt wird.

11. Verfahren nach einem der Ansprüche 9 oder 10, **dadurch gekennzeichnet, dass** das optische Signal (9) eine Mehrzahl von Wellenlängen aufweist und die Intensität des optischen Signals (9) am zweiten Ende (22) des Wellenleiters in zumindest zwei Wellenlängenbereichen bestimmt wird.

12. Verfahren nach einem der Ansprüche 9 bis 11, **dadurch gekennzeichnet, dass** die flüssige Probe auf die erste Messstelle (25) und die zweite Messstelle (45) durch folgende Schritte aufgebracht wird:
Aufbringen der flüssigen Probe auf die erste Messstelle (25) und die zweite Messstelle (45);
Abwarten einer vorgebbaren Zeitspanne;
Abwaschen der flüssigen Probe mit einem Lösungsmittel;
optionales Aufbringen einer Referenzflüssigkeit (8) zumindest auf die erste Messstelle (25) und/oder die zweite Messstelle (45).

13. Verfahren nach einem der Ansprüche 9 bis 12, **dadurch gekennzeichnet, dass** weiterhin ein optisches Signal (9) in das erste Ende (41) des zweiten Wellenleiters (4) eingekoppelt wird und die Intensität des optischen Signals (9) am zweiten Ende (42) des zweiten Wellenleiters (4) als Referenzsignal erfasst wird
und/oder
dass das Bestimmen der Intensität des optischen Signals (9) am zweiten Ende (22) der Wellenleiter (2, 4) differentiell erfolgt.

14. System mit einem Sensor (1) nach einem der Ansprüche 1 bis 8 und einem Mobiltelefon (6) oder einem Tablett-Computer.

15. System nach Anspruch 14, **dadurch gekennzeichnet, dass** der Sensor (1) ein Einwegartikel ist.

## Claims

1. Optical sensor (1) comprising a substrate (10), which has at least a first side (11) and an opposite second side (12), at least a first waveguide (2) being arranged at least on the first side (11), which has at least a first measuring point (25) which has at least one interruption (3) of the first waveguide (2), **characterized in that** the first measuring point (25) is functionalized by at least one coating (5) which allows the selective coupling of predeterminable microorganisms or subcellular pathogens,
the sensor further containing at least a second waveguide (4) which has at least a second measuring point (45), which includes at least one interruption (3) of the second waveguide (4) and is not functionalized by at least one coating.

2. Sensor according to claim 1, **characterized in that** the first measuring point (25) has between about 3 and about 60 interruptions (3) or **in that** the first measuring point (25) has between about 10 and about 30 interruptions (3) or **in that** the first measuring point (25) has between about 5 and about 20 interruptions (3) or **in that** the first measuring point (25) has between about 50 and about 100 interruptions (3).

3. Sensor according to claim 1 or 2, **characterized in that** the interruptions (3) of the first waveguide (2) are arranged periodically in the first measuring point (25)
or
**in that** the interruptions (3) of the first waveguide (2) in the first measuring point (25) have a length of about 2 µm to about 20 µm or
a length of about 3 µm to about 10 µm or
a length of about 2 µm to about 6 µm.

4. Sensor according to any of claims 1 to 3, **characterized in that** the waveguide (2) has a first end (21) and an opposite second end (22), one coupler each being disposed at the first end (21) and/or at the second end (22).

5. Sensor according to any of claims 1 to 4, **characterized in that** the substrate contains, or consists of, a glass or a polymer or a semiconductor.

6. Sensor according to any of claims 1 to 5, further containing at least a third waveguide which has a first end and an opposite second end, wherein the second end is designed to be coupled to a detector (62) and the first end of the third waveguide is led to the first measuring point (25) so that scattered light from the first measuring point (25) can be coupled into the third waveguide.

7. Sensor according to any of claims 1 to 6, further containing at least a fourth waveguide which has a first end and an opposite second end, wherein the second end is designed to be coupled to a detector (62) and the first end of the fourth waveguide is led to the second measuring point (45) so that scattered light from the second measuring point (45) can be coupled into the fourth waveguide.

8. Sensor according to any of claims 1 to 7, further containing at least one light source (61) and/or at least one detector (62), wherein the light source (61) can be connected to the first end (21) of the waveguide (2) and the detector (62) can be connected to the second end (21) of the waveguide (2).

9. Method for detecting pathogenic germs, comprising the following steps:
providing an optical sensor (1) with a substrate (10), which has at least a first side (11) and an opposite second side (12), wherein at least on the first side (11) at least a first waveguide (2) is arranged, which has a first end (21) and an opposing second end (22) and at least a first measuring point (25), which contains at least one interruption (3) of the first waveguide (2) and wherein the first measuring point (25) is functionalized by at least one coating (5), wherein the coating allows the selective coupling of predeterminable microorganisms or subcellular pathogens, and the sensor further contains at least a second waveguide (4) which has a first end (21) and an opposite second end (22) and at least a second measuring point (45) which has at least one interruption (3) of the second waveguide (4) and is not functionalized by at least one coating;
applying a liquid sample which contains pathogenic germs (75) at least to the first measuring point (25);
coupling an optical signal (9) into the first end (21) of the first waveguide (2);
determining the intensity of the optical signal (9) at the second end (22) of the first waveguide (2).

10. Method according to claim 9, **characterized in that** the optical signal (9) has a single wavelength and the intensity of the optical signal (9) is determined with this wavelength at the second end (22) of the waveguide.

11. Method according to any of claims 9 or 10, **characterized in that** the optical signal (9) has a plurality of wavelengths and the intensity of the optical signal (9) is determined at the second end (22) of the waveguide in at least two wavelength ranges.

12. Method according to any of claims 9 to 11, **characterized in that** the liquid sample is applied to the first measuring point (25) and the second measuring point (45) by the following steps:
applying the liquid sample to the first measuring point (25) and the second measuring point (45);
waiting for a predeterminable period of time;
washing off the liquid sample with a solvent;
optionally applying a reference liquid (8) at least to the first measuring point (25) and/or the second measuring point (45).

13. Method according to any of claims 9 to 12, **characterized in that** an optical signal (9) is further coupled into the first end (41) of the second waveguide (4) and the intensity of the optical signal (9) at the second end (42) of the second waveguide (4) is detected as a reference signal and/or
**in that** the intensity of the optical signal (9) is determined differentially at the second end (22) of the waveguides (2, 4).

14. System comprising a sensor (1) according to any of claims 1 to 8 and a cell phone (6) or a tablet computer.

15. System according to claim 14, **characterized in that** the sensor (1) is a disposable article.

## Revendications

1. Capteur optique (1) comprenant un substrat (10) qui présente au moins une première face (11) et une deuxième face opposée (12), au moins un premier guide d'ondes (2) étant disposé au moins sur la première face (11), lequel présente au moins un premier point de mesure (25) qui présente au moins une interruption (3) du premier guide d'ondes (2),
**caractérisé en ce que**
le premier point de mesure (25) est fonctionnalisé par au moins un revêtement (5) qui permet le couplage sélectif de micro-organismes prédéfinis ou d'agents pathogènes subcellulaires,
le capteur comprenant en outre au moins un deuxième guide d'ondes (4) qui présente au moins un deuxième point de mesure (45) qui présente au moins une interruption (3) du deuxième guide d'ondes (4) et qui n'est pas fonctionnalisé par au moins un revêtement.

2. Capteur selon la revendication 1,
**caractérisé en ce que** le premier point de mesure (25) présente entre environ 3 et environ 60 interruptions (3), ou
**en ce que** le premier point de mesure (25) présente entre environ 10 et environ 30 interruptions (3), ou
**en ce que** le premier point de mesure (25) présente entre environ 5 et environ 20 interruptions (3), ou
**en ce que** le premier point de mesure (25) présente entre environ 50 et environ 100 interruptions (3).

3. Capteur selon la revendication 1 ou 2,
**caractérisé en ce que** les interruptions (3) du premier guide d'ondes (2) sont disposées de manière périodique dans le premier point de mesure (25), ou
**en ce que** les interruptions (3) du premier guide d'ondes (2) dans le premier point de mesure (25) ont une longueur d'environ 2 µm à environ 20 µm, ou
une longueur d'environ 3 µm à environ 10 µm, ou
une longueur d'environ 2 µm à environ 6 µm.

4. Capteur selon l'une des revendications 1 à 3,
**caractérisé en ce que** le guide d'ondes (2) présente une première extrémité (21) et une deuxième extrémité opposée (22), un coupleur respectif se trouvant à la première extrémité (21) et/ou à la deuxième extrémité (22).

5. Capteur selon l'une des revendications 1 à 4,
**caractérisé en ce que** le substrat comprend ou est constitué d'un verre ou d'un polymère ou d'un semi-conducteur.

6. Capteur selon l'une des revendications 1 à 5,
comprenant en outre au moins un troisième guide d'ondes qui présente une première extrémité et une deuxième extrémité opposée, la deuxième extrémité étant adaptée pour être couplée à un détecteur (62), et la première extrémité du troisième guide d'ondes étant menée jusqu'au premier point de mesure (25), de sorte que la lumière diffusée du premier point de mesure (25) peut être injectée dans le troisième guide d'ondes.

7. Capteur selon l'une des revendications 1 à 6,
comprenant en outre au moins un quatrième guide d'ondes qui présente une première extrémité et une deuxième extrémité opposée, la deuxième extrémité étant adaptée pour être couplée à un détecteur (62), et la première extrémité du quatrième guide d'ondes étant menée jusqu'au deuxième point de mesure (45), de sorte que la lumière diffusée du deuxième point de mesure (45) peut être injectée dans le quatrième guide d'ondes.

8. Capteur selon l'une des revendications 1 à 7,
comprenant en outre au moins une source lumineuse (61) et/ou au moins un détecteur (62), la source lumineuse (61) pouvant être reliée à la première extrémité (21) du guide d'ondes (2), et le détecteur (62) pouvant être relié à la deuxième extrémité (21) du guide d'ondes (2).

9. Procédé de mise en évidence de germes pathogènes, comprenant les étapes suivantes consistant à :
fournir un capteur optique (1) comprenant un substrat (10) qui présente au moins une première face (11) et une deuxième face opposée (12), au moins un premier guide d'ondes (2) étant disposé au moins sur la première face (11), lequel présente une première extrémité (21) et une deuxième extrémité opposée (22) et au moins un premier point de mesure (25) qui contient au moins une interruption (3) du premier guide d'ondes (2), et le premier point de mesure (25) étant fonctionnalisé par au moins un revêtement (5), le revêtement permettant le couplage sélectif de micro-organismes prédéfinis ou d'agents pathogènes subcellulaires, et le capteur comprenant en outre au moins un deuxième guide d'ondes (4) qui présente une première extrémité (21) et une deuxième extrémité opposée (22) et au moins un deuxième point de mesure (45) qui présente au moins une interruption (3) du deuxième guide d'ondes (4) et qui n'est pas fonctionnalisé par au moins un revêtement ;
appliquer un échantillon liquide, contenant des germes pathogènes (75), au moins sur le premier point de mesure (25) ;
injecter un signal optique (9) dans la première extrémité (21) du premier guide d'ondes (2) ;
déterminer l'intensité du signal optique (9) à la deuxième extrémité (22) du premier guide d'ondes (2).

10. Procédé selon la revendication 9,
**caractérisé en ce que** le signal optique (9) présente une seule longueur d'onde, et l'intensité du signal optique (9) présentant cette longueur d'onde est déterminée à la deuxième extrémité (22) du guide d'ondes.

11. Procédé selon l'une des revendications 9 ou 10,
**caractérisé en ce que** le signal optique (9) présente une pluralité de longueurs d'onde, et l'intensité du signal optique (9) est déterminée dans au moins deux plages de longueurs d'onde à la deuxième extrémité (22) du guide d'ondes.

12. Procédé selon l'une des revendications 9 à 11,
**caractérisé en ce que** l'échantillon liquide est appliqué sur le premier point de mesure (25) et sur le deuxième point de mesure (45) par les étapes suivantes consistant à :
appliquer l'échantillon liquide sur le premier point de mesure (25) et sur le deuxième point de mesure (45) ;
attendre un laps de temps prédéfinissable ;
laver l'échantillon liquide avec un solvant ;
optionnellement, appliquer un liquide de référence (8) au moins sur le premier point de mesure (25) et/ou sur le deuxième point de mesure (45).

13. Procédé selon l'une des revendications 9 à 12,
**caractérisé en ce qu'**en outre un signal optique (9) est injecté dans la première extrémité (41) du deuxième guide d'ondes (4), et l'intensité du signal optique (9) est détectée comme signal de référence à la deuxième extrémité (42) du deuxième guide d'ondes (4), et/ou
**en ce que** la détermination de l'intensité du signal optique (9) à la deuxième extrémité (22) des guides d'ondes (2, 4) s'effectue de manière différentielle.

14. Système comprenant un capteur (1) selon l'une des revendications 1 à 8 et un téléphone mobile (6) ou une tablette informatique.

15. Système selon la revendication 14,
**caractérisé en ce que** le capteur (1) est un article à usage unique.
